(19) Europäisches Patentamt
European Patent Office
Office européen des brevets

(11) **EP 3 142 749 B1**

(12) **EUROPEAN PATENT SPECIFICATION**

(45) Date of publication and mention
of the grant of the patent:
**20.11.2019 Bulletin 2019/47**

(51) Int Cl.:
***A61K 31/728*** *(2006.01)*    ***A61K 31/738*** *(2006.01)*
***A61P 19/02*** *(2006.01)*    ***A61K 45/06*** *(2006.01)*

(21) Application number: **15726748.5**

(22) Date of filing: **18.05.2015**

(86) International application number:
**PCT/PT2015/000023**

(87) International publication number:
**WO 2015/174872 (19.11.2015 Gazette 2015/46)**

(54) **VISCOSUPPLEMENT COMPOSITION COMPRISING ULVAN FOR TREATING ARTHRITIS**

VISCOSUPPLEMENT-ZUSAMMENSETZUNG MIT ULVAN ZUR BEHANDLUNG VON ARTHRITIS

COMPOSITION DE VISCOSUPPLÉMENT COMPRENANT DES ULVANES POUR LE TRAITEMENT DE L'ARTHRITE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.05.2014 GB 201408752**

(43) Date of publication of application:
**22.03.2017 Bulletin 2017/12**

(73) Proprietor: **Stemmatters, Biotecnologia e Medicina Regenerativa SA**
**4805-017 Barco GMR (PT)**

(72) Inventors:
• **SOUSA, Rui Pedro Romero Amandi de**
**4805-017 Barco GMR (PT)**
• **GERTRUDES, Ana Catarina Freire**
**4805-017 Barco GMR (PT)**
• **CORREIA, Cristina**
**4805-017 Barco GMR (PT)**
• **MORAIS, Alain José da Silva**
**4805-017 Barco GMR (PT)**
• **GONÇALVES, Cristiana da Mota Martins**
**4805-017 Barco GMR (PT)**
• **RADHOUANI, Hajer**
**4805-017 Barco GMR (PT)**
• **GOMES, Carlos Alberto Vilela**
**4805-017 Barco GMR (PT)**
• **SANTOS, Tírcia Susete Xavier Carlos dos**
**4805-017 Barco GMR (PT)**

• **OLIVEIRA, Joaquim Miguel Antunes Correia de**
**4805-017 Barco GMR (PT)**
• **ESPREGUEIRA-MENDES, João Duarte Coelho do Sameiro**
**4805-017 Barco GMR (PT)**
• **REIS, Rui Luís Gonçalves dos**
**4805-017 Barco GMR (PT)**

(74) Representative: **Patentree**
**Edificio Net**
**Rua de Salazares, 842**
**4149-002 Porto (PT)**

(56) References cited:
**WO-A2-2006/073835    JP-A- 2009 057 285**

• **LAHAYE M ET AL: "Structure and function properties of Ulvan, a polysaccharide from green seaweeds", BIOMACROMOLECULES, AMERICAN CHEMICAL SOCIETY, US, vol. 8, no. 6, 1 June 2007 (2007-06-01), pages 1765-1774, XP002522792, ISSN: 1525-7797, DOI: 10.1021/BM061185Q [retrieved on 2007-04-26] cited in the application**
• **SAMARAWEERA A MALSHANI ET AL: "Industrial Applications of Macroalgae", 1 January 2012 (2012-01-01), HANDBOOK OF MARINE MACROALGAE : BIOTECHNOLOGY AND APPLIED PHYCOLOGY, WILEY-BLACKWELL, UK, PAGE(S) 500 - 521, XP008176948, ISBN: 978-0-470-97918-1 [retrieved on 2011-11-21] page 502 figure 33.4 page 510, right-hand column, paragraph 4 - page 512, left-hand column, paragraph 4**

**Description**

Field of the Invention

The present invention relates to an ulvan containing composition. This composition is a viscosupplement composition and can be used in the treatment of arthritis. The present invention also discloses a method of treating arthritis by administering the ulvan containing composition.

Background to the Invention

Arthritis is a chronic inflammation of the joint, which is normally accompanied by pain, swelling of surrounding connective tissue and limitation of movement. The most prevalent forms of arthritis are osteoarthritis and rheumatoid arthritis, both of which are progressive, degenerative diseases leading to varying degrees of disability. As a result of these diseases, cartilage and bone of the joint undergo progressive destruction, followed by loss of mobility and increased pain.

Osteoarthritis (OA), also designated as degenerative joint disease or arthrosis, is among the most common musculoskeletal disorders. Symptoms of OA are pain, swelling and stiffness of the articulation. Onset of OA is likely to be initiated many years prior to its clinical diagnosis, and it persists until the end stage of the disease where almost all the articular cartilage of the affected joints is lost.

Osteoarthritis is characterized by breakdown of articular cartilage due to biological or mechanical factors, which changes normal load transmission in the joint and produces pain. Inflammation of synovium may additionally cause pain, which may be triggered by cartilage particles (meniscal or articular). Cartilage degeneration stimulates production of extracellular matrix degrading enzymes and produces inflammatory mediators in the joint, which will further contribute to inflammation of synovium.

OA affects predominantly articular cartilage, which degrades by gradual loss of its extracellular matrix (ECM) composed mainly of aggrecan and type II collagen. Decrease in proteoglycan aggrecan content occurs first, resulting in a concomitant decrease in compressive stiffness. This is followed by damage to the collagen fibrillar network which is the main component responsible for tensile properties of cartilage.

Aggrecan degradation is associated with upregulation of aggrecanases, a disintegrin and metalloprotease with thrombospondin motifs (ADAMTS-) 4 and 5 as well as matrix metalloproteinases (MMPs). Excessive cleavage of type II collagen is caused by upregulation of synthesis and activity of metalloproteinases, in particular MMP-13.

Rheumatoid arthritis is an inflammatory condition with widespread synovial joint involvement. It is the most common form of chronic polyarthritis, and although it is a systemic disease, it predominantly affects peripheral joints. Persistent synovitis leads to joint destruction, which results in long-term morbidity and increased mortality. Its etiology remains unknown. Modern treatment is ineffective at controlling disease activity and reducing long-term disability, and early treatment aimed at controlling disease activity is the present prevention strategy.

Viscosupplementation consists of the injection of a gel-like, polymeric substance into the joint. The biocompatible lubricant aims at lubricating the cartilage interface, thus reducing pain and improving joint flexibility. This method of treatment usually requires several injections, as benefits are mostly temporary. Currently used substances degrade within weeks to months due to the action of enzymes in the synovial fluid which break down the polymer structure. Substances used in viscosupplementation include hyaluronic acid (HA) and poly-sulphated glycosaminoglycans (PSGAGS).

Hyaluronic acid (HA) is a natural complex sugar of the glycosaminoglycan (GAG) family. HA is a long-chain polymer containing disaccharide units of glucuronic acid and N-acetylglucosamine. HA is a naturally occurring substance found in the synovial (joint) fluid, where it acts as a lubricant and shock absorber during joint functioning. Intra-articular (IA) injections of HA have been clinically used to manage OA in patients with questionable success.

Hyaluronic acid has been widely studied for osteoarthritis application, either alone or mixed with other compounds. However, the therapeutic effect of hyaluronic acid is relatively short-lasting due to the action of the native hyaluronidase enzyme, which extensively degrades the polymer chain, leading to limited clinical efficacy. Accordingly, multiple injections of hyaluronic acid are required over time in order to maintain acceptable levels of intact hyaluronic acid at the site of action for sustained pain relief. This administration regime presents added costs and inconvenience for both the patient and physician.

Evidence from clinical studies suggests that the chondroprotective effect of HA is due to significant reduction in joint space narrowing, inhibition of matrix metalloproteinases (MMPs) in synovial tissue and reduction of MMP-9 in the synovial fluid in HA-treated patients suffering from OA.

Polysaccharides with high sulphate content are generally described as sulphated polysaccharides (SPS). SPS have gained interest in medicine due to their bioactivity and physico-chemical performance. SPS have been extensively studied for their antiviral, antitumor, antiangiogenic, anticoagulant, antioxidant, anti-inflammatory, antiproliferative, antiparasitic, antimetastatic, and immunomodulating activities.

SPS can be classified as natural (extracted from plants, animals and microorganisms) or synthetic (chemically modified or synthesized). Most mammalian SPS are GAGs, being a part of the extracellular matrix of tissues. Bioactivity of SPS depends upon the molecular weight, degree of sulphation, position of sulphate group and solubility in water.

Several SPS have been proposed as viscosupplements for the treatment of osteoarthritis. Chondroitin sulphate (CS) is a sulphated GAG composed of a chain of alternating sugars (N-acetylgalactosamine and glucuronic acid). Experimental studies have demonstrated anti-inflammatory, anabolic and anti-catabolic properties. Sulphation appears to influence the therapeutic properties of CS in osteoarthritis. CS has been referred as effective in reducing progression of joint space narrowing in patients with OA.

SPS have a high application potential in the context of OA. The intrinsic bioactive performance of SPS coupled with improved stability against enzymatic degradation compared to HA, makes these polymers potentially suitable for viscosupplement compositions. Although the use of SPS in the context of OA treatment has potential merits due to their wide bioactive spectrum and intrinsic physicochemical properties, the development of improved, longer-acting viscosupplement compositions is very challenging for those skilled in the art.

In a review by Lahaye *et al.,* (2007), it is recited that ulvan may serve as a source of rare sugars for synthesis of fine chemicals and heparin analogues. Ulvan is also suggested to be of pharmaceutical interest due to its anti-tumour, antiviral and immune modulation activities amongst others, as well as its ability to reduce serum cholesterol and triglyceride levels. Potential applications in the arthritis setting are not discussed.

Japanese patent application JP2009057285 claims the use of an angiogenesis inhibitor comprising a sulphated polysaccharide comprising rhamnose as the main component of a constituent monosaccharide or a salt thereof for the treatment of generalised disorders such as solid tumours, vascular lesions, skin, bone and joint, genital, eye and lung diseases. Such sulphated polysaccharides may be obtained from several seaweed species, including the green sea lettuce Ulva. In order to produce the technical effect, the sulphated polysaccharides must function as an angiogenesis inhibitor. The usefulness of ulvan as a viscosupplement composition acting via angiogenesis inhibition in the arthritis setting is not mentioned.

PCT application WO2006/073835 discloses viscosupplement compositions comprising hyaluronic acid for the treatment of osteoarthritis of the knee. The use of ulvan is not mentioned in this document.

## Summary of the Invention

The development of compositions for OA treatment that incorporate SPS demands the selection of an SPS taking into account many molecular parameters such as chemical structure, sulphation degree and molecular weight, among others.

The present invention addresses the need for compositions for treatment and prophylaxis of arthritis in animals, preferably in humans, that reduce the number of administrations and the respective dose per administration. The present invention relates to alternative viscosupplement compositions based on ulvan. Ulvan has not been previously used as a viscosupplement, nor has it been used in the treatment or prophylaxis of arthritis, in particular, osteoarthritis.

Therefore, in a first aspect, the present invention provides a viscosupplement composition comprising ulvan for use in the treatment, therapy or prophylaxis of a musculoskeletal disease.

In a further embodiment, the present invention provides ulvan for use in the treatment, therapy or prophylaxis of a musculoskeletal disease such as arthritis.

The invention is defined by the claims. Any subject-matter falling outside the scope of the claims is provided for information purposes only. Any references in the description to methods of treatment refer to the compounds or compositions of the present invention for use in a method for treatment of the human or animal body by therapy.

Ulvan based viscosupplements benefit from the bioactive properties of ulvan and exhibit improved degradation behaviour as compared to existing viscosupplement compositions such as hyaluronic acid.

Ulvan is a sulphated polysaccharide composed of rhamnose, xylose and uronic acids, namely glucuronic acid and iduronic acid. Ulvan contains a repeating disaccharide unit consisting of two monomers selected from rhamnose, xylose, glucuronic acid and iduronic acid. Ulvan repeating disaccharide units include at least:

    (i) [glucuronic acid-rhamnose sulphate];
    (ii) [iduronic acid-rhamnose sulphate];
    (iii) [xylose-rhamnose sulphate]; and
    (iv) [xylose sulphate-rhamnose sulphate].

Therefore, in some embodiments, the ulvan is a polymer of one or more of the above disaccharide units.

In particular, the ulvan repeating disaccharide units include:

(i) type A3S [>4-beta-D-glucuronic acid-(1>4)-alpha-L-rhamnose 3-sulphate-1>];
(ii) type B3S [>4-alpha-L-iduronic acid-(1>4)-alpha-L-rhamnose 3-sulphate-1>];
(iii) type U3S [>4-bela-D-xylose-(1>4)-alpha-L-rhamnose 3-sulphate-1>] ;
(iv) type U2'S3S [>4-beta-D-xylose 2-sulphate-(1>4)-alpha-L-rhamnose 3-sulphate-1>].

Therefore, in some embodiments, the ulvan is a polymer of one or more of the above disaccharide units.

The ulvan may be associated with salts that act as molecule stabilisers.

The ulvan used in the present invention can come from any suitable source. Ulvan can be extracted from vegetal species, for example green algae, for example as described by Alves *et al.,* 2010, although other methods for its production such as bacterial fermentation or organic synthesis can also be used.

Some or all of the monomers in ulvan are sulphated. For example, ulvan may comprise one or more of xylose sulphate, rhamnose sulphate, sulphated glucuronic acid and sulphated iduronic acid.

The sulphate content of naturally occurring ulvan varies typically between 0.1% and 25%. However, this can be increased by the partial or total sulphation of free hydroxyl groups in the ulvan, which gives rise to the respective desired sulphation degree.

In some embodiments, the sulphation degree of ulvan is 0.1% to 75%. This means that 0.1% to 75% of the hydroxyl groups of rhamnose, xylose, glucuronic acid and iduronic acid are sulphated. Preferably, the sulphation degree of ulvan is 0.1% to 40%. More preferably, the sulphation degree of ulvan is 0.1% to 30%. Even more preferably, the sulphation degree of ulvan is 0.1% to 20%. Most preferably, the sulphation degree of ulvan is 0.1% to 6%. The ulvan sulphation degree can be adjusted by chemical modification according to methods known to those skilled in the art (Papy-Garcia *et al.,* (2005) and Nagasawa *et al.,* (1977)).

In some embodiments, the ulvan has an average molecular weight between 100 and 10,000 kDa. Preferably, the ulvan has an average molecular weight between 100 and 5,000 kDa. More preferably, the ulvan has an average molecular weight between 100 and 3,000 kDa. Even more preferably, the ulvan has an average molecular weight between 100 and 1,200 kDa. Most preferably, the ulvan has an average molecular weight between 100 and 600 kDa.

Naturally occurring ulvan has a relatively high average molecular weight. If the molecular weight is regarded as too high, lower molecular weight molecules like oligosaccharides can be derived from ulvan by hydrolysis of the starting molecule. Preferably, the lower molecular weight molecules derived from ulvan are in the range of 30 to 100 kDa. If necessary, the lower molecular weight ulvan molecules can be covalently crosslinked to increase the molecular weight. Preferably, the crosslinked ulvan molecules have an average molecular weight between 100 and 10,000 kDa. Adjustment of the degree of sulphation of ulvan can be performed before and/or after the hydrolysis step. In some embodiments, the oligosaccharides produced by the hydrolysis have a molecular weight below 100 kDa.

In some embodiments, the ulvan comprises 5% to 50% glucuronic acid, and 0 to 50% iduronic acid, in terms of the relative molar percentage of monomers. Preferably, the ulvan comprises 15% to 50% glucuronic acid, and 0 to 30% iduronic acid, in terms of the relative molar percentage of monomers. More preferably, the ulvan comprises 25% to 50% glucuronic acid, and 0 to 15% iduronic acid, in terms of the relative molar percentage of monomers.

In some embodiments, the ulvan comprises 25% to 100% of the disaccharide unit [glucuronic acid-rhamnose sulphate] and 25% to 100% of the disaccharide unit [iduronic acid-rhamnose sulphate], in terms of the relative molar percentage of units. Preferably, the ulvan comprises 40% to 100% of the disaccharide unit [glucuronic acid-rhamnose sulphate] and 25% to 70% of the disaccharide unit [iduronic acid-rhamnose sulphate], in terms of the relative molar percentage of units. More preferably, the ulvan comprises 40% to 100% of the disaccharide unit [glucuronic acid-rhamnose sulphate] and 25% to 50% of the disaccharide unit [iduronic acid-rhamnose sulphate], in terms of the relative molar percentage of units.

In particular embodiments, ulvan comprises 25% to 100% of the A3S: and 25% to 100% of the B3S repeating disaccharide unit, in terms of the relative molar percentage of units. Preferably, the ulvan comprises 40% to 100% of the A3S and 25% to 70% of the B3S repeating disaccharide unit, in terms of the relative molar percentage of units. More preferably, the ulvan comprises 40% to 100% of the A3S and 25% to 50% of the B3S repeating disaccharide unit, in terms of the relative molar percentage of units.

Ulvan is soluble in aqueous solutions, and is insoluble in almost all organic solvents. Ulvan molecule surface charge depends on solution pH and ionic strength. Changes in solution osmolality and pH, and in ulvan concentration, result in solutions with different viscosity. Ulvan has the ability to form weak gels in the presence of divalent cations, such as boron and calcium (Lahaye *et al.,* 2007).

In the context of this invention, ulvan functional groups, such as hydroxyl, carboxyl and sulphate groups, can be used to incorporate additional functional groups like methacrylate, amine, aldehydes, itaconates, acrylamides, or acrylates. In this regard, the degree of substitution of the ulvan functional groups may be between 0.1% and 50%. In a preferred embodiment, the degree of substitution of the ulvan functional groups is between 0.1% and 40%, preferably below 30%.

The composition is preferably in liquid form. Preferably, the composition is an aqueous composition. Preferably, the ulvan has a concentration of 0.1% to 30% w/V in the composition. In some embodiments, the ulvan has a concentration of 0.2% to 20% w/V in the composition. In other embodiments, the ulvan has a concentration of 0.3% to 15% w/V in the composition. In particular embodiments, the ulvan has a concentration of 0.4% to 12% w/V in the composition. In certain embodiments, the ulvan has a concentration of 0.5% to 10% w/V in the composition.

The composition may comprise a buffer to help to maintain the composition at a particular pH. The buffer may be any suitable buffer, for example, a phosphate buffer or a Tris-HCl buffer.

The composition may have a pH of 4 to 10. In some embodiments, the composition has a pH of 5 to 9. In other embodiments, the composition has a pH of 6 to 8. In further embodiments, the composition has a pH of 6.8 to 7.4. In particular embodiments, the composition has a pH of about 7.

In some embodiments, the composition comprises physiological saline solution or cell culture media.

The composition may comprise one or more sulphated polysaccharides in addition to the ulvan. Preferably, the sulphated polysaccharides are selected from the group consisting of gellan sulphate, chondroitin sulphate, keratan sulphate, heparin sulphate, dextran sulphate and xylose sulphate. These are selected according to the following order of preference (starting with the most preferred): chondroitin sulphate, gellan sulphate, xylose sulphate, dextran sulphate, keratan sulphate, and heparin sulphate.

In some embodiments, the composition further comprises hyaluronic acid.

In some embodiments, the composition is totally or partially in acid form. Alternatively, the composition (in particular the acid groups) may be totally or partially in salt form. The salt should be a physiologically acceptable salt. The salt may be formed with an alkali or alkaline earth metal, such as $Na^+$, $K^+$, $Sr^+$, $Ca^{2+}$, $Ba^{2+}$, $Mg^{2+}$, or with organic compounds such as primary amines, secondary amines or tertiary amines.

The composition may comprise 0.1% to 10% w/V of a salt. In some embodiments, the composition comprises 0.2% to 8% w/V of a salt. In other embodiments, the composition comprises 0.3% to 7% w/V of a salt. In various embodiments, the composition comprises 0.4% to 6% w/V of a salt. In particular embodiments, the composition comprises 0.5% to 5% w/V of a salt. The salt may be any suitable salt as suggested above. In some embodiments, the salt is NaCl.

In various embodiments, the composition comprises additional salts such as $CuSO_4$ and/or $H_3BO_4$.

The compositions described above can be used in the preparation of pharmaceutical compositions to be used in the treatment and prophylaxis of osteoarthritis. The compositions can be combined with other excipients or active substances used in the context of veterinarian and human medicine.

The compositions can be administered by various routes, including topical, enteral and parenteral. Parenteral administration routes include intra-arterial, intra-articular, intracavitary, intradermal, intralymphatic, intramuscular, intra-synovial, intravenous, and subcutaneous. Enteral routes include oral and gastro-intestinal. Topical routes include application into the skin and mucous membranes.

In a preferred embodiment, the composition is delivered to a patient by intra-articular injection into a diseased joint, which can be repeated according to a clinical prescription regime. In an even more preferred embodiment, the composition is delivered by a single intra-articular injection into a diseased joint.

Dosage of the composition can be adapted to the administration route, as well as to the patient profile, including age, gender, condition, disease progression, or any other phenotypic or environmental parameters.

The composition may be in a solid form such as an amorphous, crystalline or semicrystalline powder, granules, flakes, pills, scaffolds, capsules and suppositories. Such a solid form can be converted into a liquid form by mixing the solid with a physiologically appropriate liquid such as solvents, solutions, suspensions and emulsions.

In another disclosure, the present invention provides a method of treating a patient suffering from a musculoskeletal disease, the method comprising administering an effective amount of the composition described above to the patient.

As described above, the composition may be administered by intra-articular injection, for example, into a joint of the patient. The joint may be an arthritic joint.

The musculoskeletal disease may be arthritis. The arthritis may be osteoarthritis arthritis (arthrosis) or rheumatoid arthritis. In some embodiments, the patient is suffering from rheumatoid arthritis. In other embodiments, the patient is suffering from osteoarthritis.

In a further aspect, the present invention provides ulvan for use in the treatment or prophylaxis of a musculoskeletal disease, for example, arthritis. Further, the present invention discloses the use of ulvan in the manufacture of a medicament for the treatment or prophylaxis of a musculoskeletal disease, for example, arthritis.

Brief Description of the Figures

The invention will how be described in detail, by way of example only, with reference to the following Figures:

Figure 1. Schematic representation of four possible repetition units of ulvan: two aldobiouronic acids (A3S and B3S), and two ulvanobioses (U3S and U2S3S).

Figure 2. $^1$H NMR spectrum at 60 °C of ulvan dissolved in deuterium oxide.

Figure 3. $^1$H NMR spectrum at 60 °C of glucuronic acid dissolved in deuterium oxide.

Figure 4. $^1$H NMR spectrum at 60 °C of rhamnose dissolved in deuterium oxide.

Figure 5. $^1$H NMR spectrum at 60 °C of xylose dissolved in deuterium oxide.

Figure 6. $^1$H NMR spectrum at 70 °C of methacrylated ulvan dissolved in deuterium oxide. Arrow indicates the peaks of new protons in ulvan backbone which results from methacrylation reaction.

Figure 7. Image of the hydrogels produced from methacrylated ulvan after 10 minutes UV-light irradiation. Left: front image of the hydrogel; Right: top image of the hydrogel.

Figure 8. Gene expression ratio of collagen type II, collagen type I and collagen type X after treatment, normalized to non-treated group.

Figure 9. Microscopic imaging (5x) of rabbit articular cartilage sections stained with safranin O. Left: Staining of normal articular cartilage and subchondral bone. Middle: Staining of articular cartilage after 3 weeks of osteoarthritis (OA) induction. Right: Staining of osteoarthritic articular cartilage after 8 weeks of treatment with ulvan formulation (3% w/V).

Detailed Description of the Invention

The following examples are merely illustrative and should not be construed to limit the scope of the disclosure.

Example 1: Characterization of ulvan salts

Ulvan salt was prepared in different solvents for chemical characterization.

**Methods & Results**

**Monomeric composition**

Ulvan was solubilized in $H_2O$ at room temperature, and then hydrolysed with $H_2SO_4$ 1 M at 100°C for 2.5 hours. Neutral sugars were determined as alditol acetates using gas chromatography analysis as described by Coimbra *et al.* (1996). Uronic acids were determined by an adapted 3-phenylphenol colorimetric method described by Coimbra *et al.* (1996). Linkage analysis was executed by methylation as adapted by Coimbra *et al.* (1996). Results are shown in Table 1.

**Repetition unit and sulphate content**

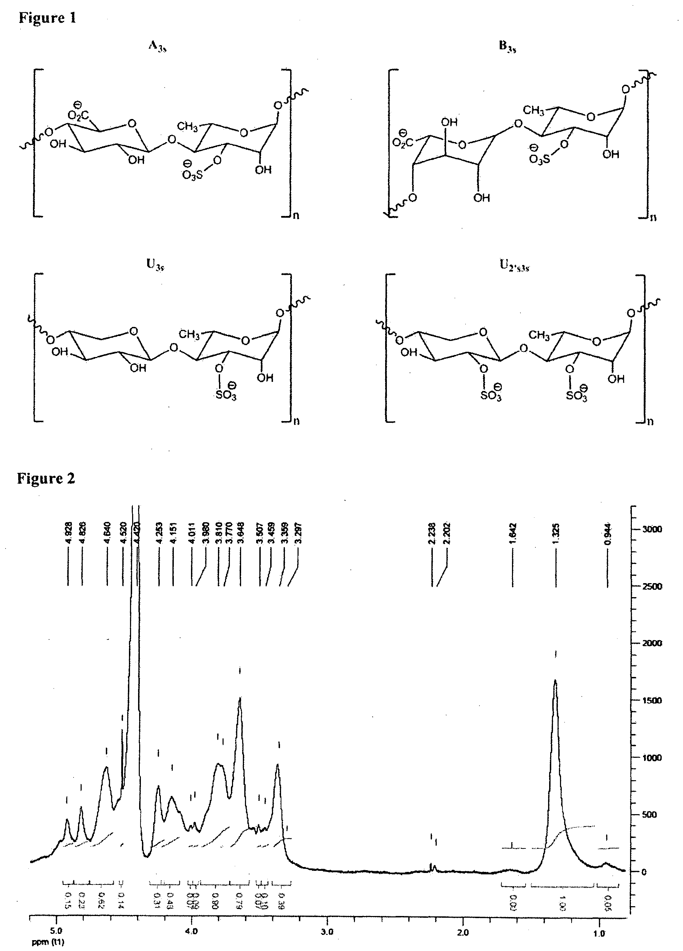

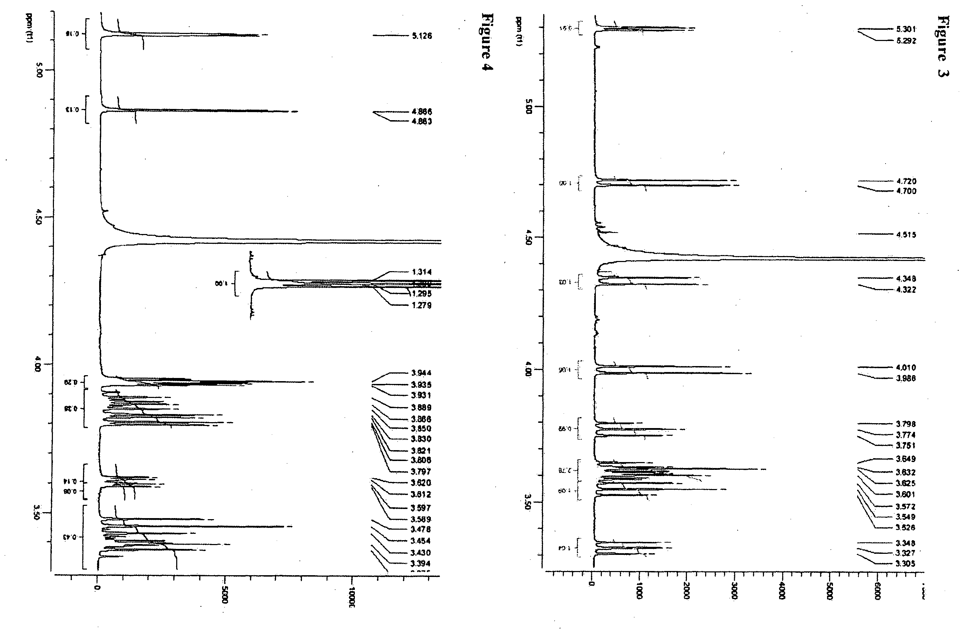

Ulvan (I % w/V) was solubilized in deuterium oxide at room temperature. Sample was analysed by $^1$H NMR acquired on a Varian Unity Plus (Varian, USA) spectrometer at 60°C (Figure 2).
$^1$H-NMR spectra were also obtained for the monomers mostly present within ulvan composition (glucuronic acid, rhamnose and xylose, Figures 3-5). These monomers were prepared at 1% w/V solution in deuterium oxide, as for the ulvan solution. The chemical shifts of the main repetition unit of the produced ulvan were identified based on the literature (Barros *et al.,* 2013; Lahaye, Inizan, & Vigouroux, 1998; Robic, Sassi, & Lahaye, 2008). The intensity of rhamnose and glucuronic acid peaks was compared with the remaining peaks of the spectra, in order to identify the relative percentage of the repetition unit, such as the aldobiouroninc A3S (>4-beta-D-glucuronic acid-(1>4)-alpha-L-rhamnose 3-sulphate-1>). By the relative composition of monomers and analysis of their linkage, the relative composition of aldobiouronic and ulvanobiose repetition unit can also be determined. Results are shown in Table 1.
Sulphate content of ulvan sample was calculated based on $^1$H NMR spectrum analyses assuming that: (i) all the rhamnose units are sulphated; and (ii) there exists one unit of rhamnose per glucuronic acid. Considering these assumptions, the calculation is made by the following developed Equation 1. Results are in Table 1.

$$\% \ S = \frac{w_S}{w_{total}} \times 100 = \frac{\frac{Mw_S \times A_{RhmS}}{Mw_{RhmS}} \times Mw_S}{Mw_{RhmS} \times A_{RhmS} + Mw_{GA} \times A_{GA}} \times 100 = \frac{26.35 \times A_{RhmS}}{243.22 \times A_{RhmS} + 194.14 \times A_{GA}} \times 100$$

Equation 1 - Equation developed to calculate ulvan sulphate percentage using $^1$H NMR spectrum. Where ws: sulfur weight; $w_{total}$: total weight; Mw: molecular weight; A:anomeric C peak integration: RhmS: sulphated rhamnose; GA: glucuronic acid; S:sulfur

**Estimated sulphate degree**

Based on sulphate content and molecular weight of ulvan, the sulphation degree was estimated using equation 2. Results are in Table 1.

$$S_T = \frac{Mw \times S\%}{100}$$

$$n_S = \frac{S_T}{Mw_S}$$

$$O_T = n_S \times 3 \times Mw_O$$

$$OH_T = \left(\frac{Mw - S_T - O_T}{Mw_A}\right) \times 4$$

$$SD = \left(\frac{n_S}{OH_T}\right) \times 100$$

Equation 2 - Equation developed to estimate ulvan sulphation degree (SD). Where $S_T$: Total sulfur mass in the molecule; Mw: molecular weight of ulvan; S%: sulphate content of ulvan, ns: estimated number of sulfur atoms; Mws: Sulfur molecular weight; $O_T$: Total oxygen mass in the molecule; $Mw_O$: Oxygen molecular weight; $OH_T$: Total number of hydroxyl groups in the molecule; $Mw_A$: Molecular weight of a repeating unit without sulfur; Note that molecular weight of A3S and B3S repeating units are equivalent, equation applies equally to both.

**Molecular weight**

Ulvan was solubilized in NaCl 0.3 M (eluent solution) at a final concentration of 0.1 % w/V. The solution was analysed by gel permeation chromatography in the equipment Viscotek TDA 305, with the three detectors: light scattering, refractive index and viscometer. Column set was composed by a guard pre-column Aq. Guard (Viscotek) and a PLaquagel-OH Mixed 8 μm (Polymer Laboratories). Elution was performed at 30 °C using a flow rate of 1 ml/min. Triple detection calibration using polyethylene oxide as narrow standard was performed for molecular weight (Mw) calculation. Results are in Table 1.

Table 1 - Chemical characterization of ulvan samples

| Ulvan sample | Monosaccharide (mol %) | | | Aldobiouronic unit (A3S and B3S) (%) | Sulphate (%) | Mw (KDa) | SD |
|---|---|---|---|---|---|---|---|
| | Rhamnose | Glucuronic acid | Xylose | | | | |
| Ulvan CRD | - | - | - | - | 4.60 ±0.11 | 1045 ± 102 | 14.5% |

(continued)

| Ulvan sample | Monosaccharide (mol %) | | | Aldobiouronic unit (A3S and B3S) (%) | Sulphate (%) | Mw (KDa) | SD |
|---|---|---|---|---|---|---|---|
| | Rhamnose | Glucuronic acid | Xylose | | | | |
| Ulvan PRFD | 29.1 ±0.3 | 23.2 ±0.1 | 33.6 ±0.8 | ≈ 75 | 4.99 ±0.37 | 639 ± 108 | 15.9% |
| Ulvan 121314 | - | - | - | ≈ 90 | 5.00 ±0.15 | 266 ± 19 | 16.0% |

Ulvan samples obtained from different processes were characterized for its chemical properties. Notorious differences were registered between ulvan samples, especially on the average molecular weight (Mw). Additionally, the composition of the tested samples is also remarkably distinct, whereas aldobiouronic units were the most present in analysed samples. This fact indicates that ulvan 121314, with 90% aldobiouroninc, possibly A3S by $^1$H NMR analysis, is expected to present less than 5 mol % of the monosaccharide xylose, whereas the ulvan PRFD presents almost 30 mol %.

Example 2: Degradation of ulvan by cartilage degradative enzymes

Osteoarthritic joints are characterized for expression of enzymes that degrade the cartilage matrix components, namely hyaluronidase. Additionally, hyaluronidase promotes rapid degradation of hyaluronic acid based viscosupplements accelerating its clearance.

**Methods**

**Resistance to hyaluronidase**

Ulvan was dissolved in phosphate buffer saline (PBS) at room temperature at the final concentrations of 0.5, 1.0 and 3.0 % w/V. Hyaluronic acid (HA) was dissolved in phosphate buffer saline at room temperature for the final concentrations of 0.1, 0.5 and 1.0 % w/V. Solutions were incubated in optimal conditions (pH 7, 37°C, 48 hours, 75 rpm) with hyaluronidase (EC 3.2.1.35). Solutions were then evaluated for polysaccharide molecular weight alteration by gel permeation chromatography as described above. Results are shown in Table 2.

**Results**

Table 2 - Molecular weight of ulvan and hyaluronic acid after 48 hours of incubation with hyaluronidase in optimal conditions. Samples incubated without enzyme were used as control.

| Sample | Hyaluronidase (units/mL) | Mw (KDa) |
|---|---|---|
| Ulvan 121314 | 0 | 266 ± 19 |
| | 300 | 278 ± 12 |
| Hyaluronic acid | 0 | 589 ± 15 |
| | 300 | 27 ± 17 |

HA molecular weight was reduced by 97.3 ±1% by incubation with hyaluronidase, although ulvan had no molecular weight alteration. These results demonstrate that ulvan is resistant to hyaluronidase degradation in optimal conditions and hyaluronidase action over HA is dramatic. Given that HA viscosity is described in the literature as being associated with its high molecular weight, the degradation power of hyaluronidase over HA is likely to reduce HA efficiency as a viscosupplement. The greater stability of ulvan towards hyaluronidase means that ulvan will remain intact over a longer timeframe than hyaluronic acid and as a consequence, lower dosages and fewer injections of ulvan should be required for effective pain relief.

Example 3: Anti-oxidant and anti-coagulant potential of ulvan

Osteoarthritic joints present an oxidative environment, which is associated with inflammation and pain. Additionally, parenteral administration of a viscosupplement, for example by intra-articular injection, presents high risk of bloodstream contact, for which it is important to avoid potential induction of blood clot formation. This example demonstrates the reducing power capacity of ulvan and proves that ulvan is non-thrombogenic, which is relevant for its potential application as an injectable formulation.

**Methods**

Ulvan samples and hyaluronic acid were dissolved at 0.5 % w/V in the mandatory solvent for each analysis performed. Hyaluronic acid is the gold standard for osteoarthritis therapy, therefore it was used in this example as a basis for comparison. Heparin is a well-known standard for anti-coagulant activity tests. The reducing power of samples was quantified by the following protocol. Samples were prepared in PBS and mixed with potassium ferricyanide, then heated at 50 °C for 20 min. The reaction was stopped by the addition of trichloroacetic acid solution (10% w/V). The solution was centrifuged and supernatant mixed with distilled water and ferric chloride (0.1% w/V). Absorbance was measured at 700 nm. Ascorbic acid was used as standard for reducing power. Results are in Table 3.
Anti-coagulant activity was quantified using heparin as a reference substance. Measurement of prothrombin time (PT) and activated partial thrombiplastin time (aTPP) was performed as previously described by Subhapradha *et al.* Results are shown in Table 4.

**Results**

Table 3 - Reducing power capacity of ulvan samples and hyaluronic acid with ascorbic acid as standard

| Sample (0.5 % w/V) | Reducing power % |
| --- | --- |
| Ulvan 121314 | 35 $\pm$ 4 |
| Hyaluronic acid | 3 $\pm$ 0 |

Ulvan presented 35% reducing power compared to ascorbic acid. Hyaluronic acid tested under the same conditions did not provide significant reducing power. This result indicates that ulvan can act as a potential anti-oxidant agent in osteoarticular environments unlike hyaluronic acid.

Table 4 - Anti-coagulant activity of ulvan evaluated by PT and aPTT methods

| Blood treatment | PT (s) | aPTT (s) |
| --- | --- | --- |
| None | 10.0 | 22.5 |
| 0.5% w/V ulvan in PBS | 10.6 | 81.1 |
| 0.05% w/V heparin in PBS | 14.4 | 364.6 |

Ulvan presents anti-coagulant activity, by slightly increasing the coagulation time in PT test and very significantly in aPTT test. When compared to heparin, it is possible to verify that ulvan's anti-coagulant activity is not as high as heparin. This result indicates that ulvan will not promote an ischemic response, which is highly relevant taking into account its possible administration by parenteral routes.
These results indicate that ulvan can act as anti-oxidant agent in osteoarticular environments without eliciting a thrombogenic response.

Example 4: Chemical modification of ulvan

Every ulvan repetition unit presents at least one reactive group: hydroxyl, carboxyl and sulphated group. These available groups allow further chemical modification of ulvan polysaccharide.

**Methods**

Methacrylated ulvan was manufactured by application of the method described in WO 2011/119059 A1. Methacrylated ulvan, the reaction product, was solubilized in deuterium oxide at room temperature. The sample was analysed by $^1$H NMR acquired on a Varian Unity Plus (Varian, USA) spectrometer at 70°C. Substitution degree was calculated based on the method from M. Hamcerencu M. (2008)..

Methacrylated ulvan was dissolved in Tris-HCl 2 M and $H_3BO_4$ 40 mM for final concentration of 4% w/V. Photoinitiator methyl benzoylformate (MBF) was added. The solution was exposed to UV-light and methacrylated ulvan hydrogels were produced.

**Results**

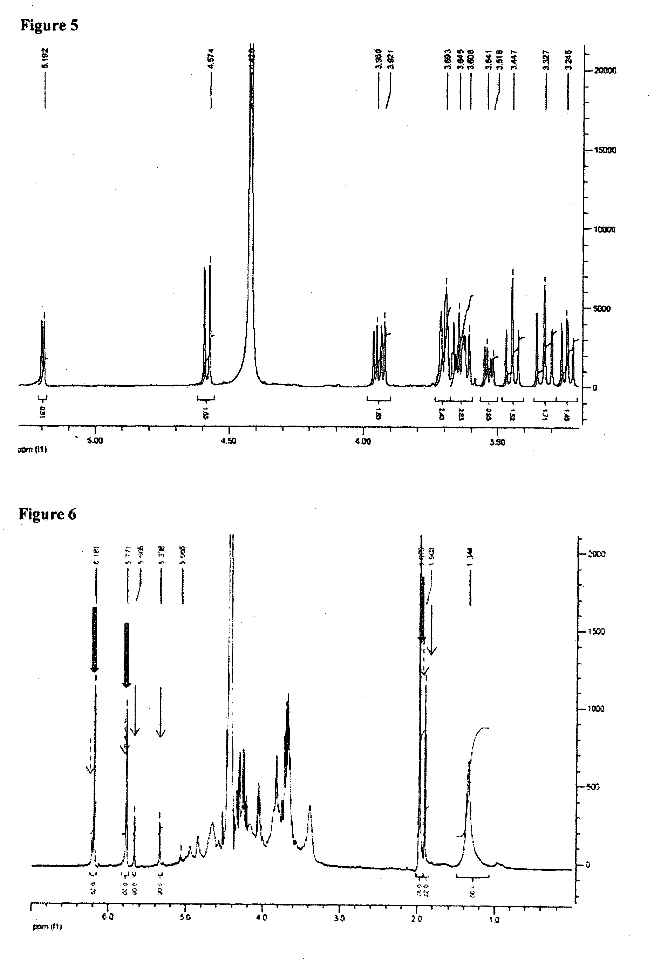

Figure 6 represents a $^1$H NMR spectrum at 70 °C of reaction product dissolved in deuterium oxide. The signals in the spectral region of 5.33-6.18 ppm were attributed to the vinyl carbon-linked hydrogen (C=CH2). The peaks shown at 1.90-1,97 ppm, were ascribed to methyl groups adjacent to the double bond (CH3-C=CH2). These signals indicate the successful formation of methacrylated ulvan. Methacrylated ulvan substitution degree was 17 % $\pm$1.

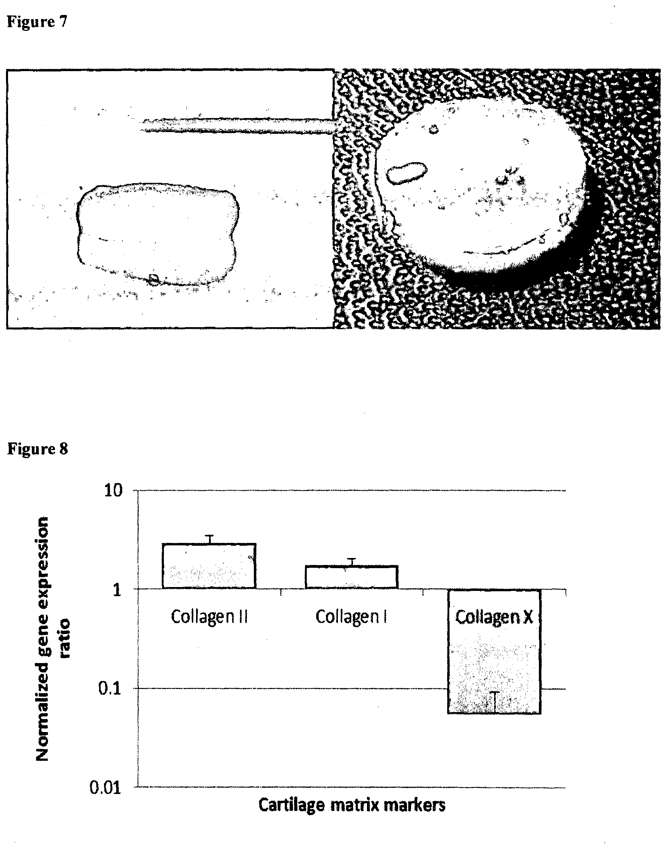

Figure 7 illustrates methacrylated ulvan hydrogels produced by photo-crosslinking. Hydrogels present the shape of the used mold and a yellowish coloration, which is typical of ulvan solutions.

Example 5: Evaluation of the *in vitro* effect of ulvan formulations on a primary culture of human osteoarthritic articular chondrocytes

This procedure may be applied to the evaluation of any ulvan formulation according to the invention.

**Methods**

Human articular cartilage was obtained from patients diagnosed with osteoarthritis and undergoing total knee replacement. Chondrocytes were isolated and cultured following methods described by Masuda and Sah (2006). Osteoarthritic chondrocytes were exposed to ulvan formulation (Z014, see table 5) (treated group), or culture media only (untreated group) for 24h. Cells were further collected for gene expression of collagen type II, collagen type I and collagen type X, by quantitative real time polymerase chain reaction (qRT-PCR). Gene expression of treated group was normalised to untreated control group, and presented as normalised expression ratio, according to Livak and Schmittgen (2011). Data is presented as average $\pm$SD.

**Results**

Figure 8 represents the normalised expression ratio of three genes quantified by real time PCR (qRT-PCR) - genes coding for collagen type II, collagen type I and collagen type X proteins. After treatment, ulvan formulation induced a 2.9x increase in the expression of collagen type II, relatively to untreated cells, marker of healthy cartilage matrix. Regarding expression of proteins related to unhealthy cartilage matrix, while collagen type I was upregulated 1.7x, collagen type X was downregulated 0.06x relatively to untreated cells. This trend indicates thatexposure of OA chondrocytes to ulvan formulation most notably promotes gene expression related with collagen type II expression, which is a sign of healthy extracellular expression.

Example 6: Evaluation of the *in vivo* effect of ulvan formulations on rabbit osteoarthritic articular cartilage model

This procedure may be applied to the evaluation of any ulvan formulation according to this invention.

**Materials & Methods**

A rabbit osteoarthritis model was used to test the effects of ulvan formulations (A132, see table 5) on the progression of OA. An osteoarthritic condition was induced in the animal's knee by medial meniscectomy following methods described by Smith and Little (2007). An 8 week treatment was tested by periodic intra-articular injection of ulvan formulation every 2 weeks. After treatment, articular cartilage samples were harvested for histological analysis. Safranin O/fast green staining was performed to identify status of cartilage matrix.

**Results**

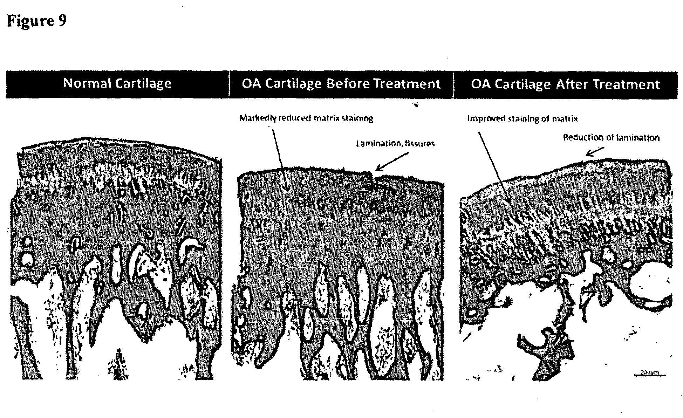

Figure 9 represents microscopic images of rabbit articular cartilage sections stained with safranin O/fast green. The left image presents staining of normal articular cartilage (stained red) and subchondral bone (stained blue-green). The middle image demonstrates staining of articular cartilage immediately after osteoarthritis induction. Signs of OA are revealed such as delamination and fibrillation of the cartilage surface, fissures within the matrix as well as glycosaminoglycan loss within the tissue. The right image presents staining of osteoarthritic articular cartilage after 8 weeks of treatment with ulvan formulation (3% w/V). Visible improvement of the physical condition of the articular cartilage is observed: stronger staining of the cartilage matrix as well as lower fibrillation and fissures.

Example 7 - Ulvan Formulations

Ulvan formulations are prepared under aseptic environment. Ulvan in powder form can be dissolved in an appropriate liquid. Table 5 presents ulvan formulations and their composition.

**Preparation of ulvan formulation A132**

Under aseptic environment, 30 mg of ulvan in solid form is dissolved in 1 mL of sterile phosphate buffer saline, pH 7. Dissolution occurs at room temperature in 30 minutes under rotational agitation.

Table 5 - Composition of ulvan formulations

| Ulvan formulation ID | Ulvan concentration (% w/V) | Liquid | Additional components |
|---|---|---|---|
| A125 | 0.5 | phosphate buffered saline, pH 7 | None |
| A128 | 1.0 | | |
| A132 | 3.0 | | |
| A138 | 10 | | |
| B125 | 0.5 | phosphate buffered saline, pH 7 | Hyaluronic acid |
| B128 | 1.0 | | |
| B132 | 3.0 | | |
| B138 | 10 | | |
| E125 | 0.5 | 0.9% w/V NaCl, pH 7 | None |
| E128 | 1.0 | | |
| E132 | 3.0 | | |
| E138 | 10 | | |
| E155 | 0.5 | 0.9% w/V NaCl, pH 7 | Hyaluronic acid |
| E159 | 1.0 | | |
| E163 | 3.0 | | |
| E175 | 10 | | |
| E202 | 0.5 | Tris-HCl buffer, pH 7 with $CuSO_4$ and $H_3BO_4$ | none |
| E203 | 1.0 | | |
| E205 | 3.0 | | |
| E212 | 10 | | |
| Z014 | 3.0 | Cell culture media | None |

References

Alves, A., Caridade, S. G., Mano, J. F., Sousa, R. A. & Reis, R. L. (2010) Extraction and physico-chemical characterization of a versatile biodegradable polysaccharide obtained from green algae. Carbohydrate Research 345: 2194-2200. doi:10.1016/j.carres.2010.07.039

Barros, A. A. A., Alves, A., Nunes, C., Coimbra, M. A., Pires, R. A. & Reis, R. L. (2013). Carboxymethylation of ulvan and chitosan and their use as polymeric components of bone cements. Acta Biomaterialia, (July) 1-12. doi:10.1016/j.actbio.2013.06.036

Coimbra, M. A., Delgadillo, I., Waldron, K. W. & Selvendran, R. R. (1996). Isolation and Analysis of Cell Wall Polymers from Olive Pulp. In H.-F. Linskens & J. F. Jackson. Modern Methods of Plant Analysis (pp. 19-44). Berlin: Springer-Verlag.

Lahaye, M., Inizan, F. & Vigouroux, J. (1998). NMR analysis of the chemical structure of ulvan and of ulvan-boron complex formation. Carbohydrate Polymers, 36, 239-249.

Robic, A., Sassi, J.-F. & Lahaye, M. (2008). Impact of stabilization treatments of the green seaweed Ulva rotundata (Chlorophyta) on the extraction yield, the physico-chemical and rheological properties of ulvan. Carbohydrate Polymers, 74(3), 344-352. doi:10.1016/j.carbpol.2008.02.020

Gonçalves, C., Rodriguez-Jasso, R. M., Gomes, N., Teixeira, J. A. & Belo, I. (2010). Adaptation of dinitrosalicylic acid method to microtiter plates. Analytical Methods, 2(12), 2046

Subhapradha N., Suman S., Ramasamy P., Saravanan R., Shanmugan V., Srinivasan A. & Shanmugan A. (2013) Anticoagulant and antioxidant activity of sulphated chitosan from the shell of donacid clam Donax scortum (Linnaeus, 1758); International Journal of Nutrition, Pharmacology, Neurological Diseases. 3(1):39-45.

Hamcerencu, M., Desbrieres, J., Khoukh A., Popa M. & Riess G. (2008) Synthesis and characterization of new unsaturated esters of gellan gum. Carbohydrate Polymers, 71, 92-100.

Masuda, K & Sah RL in "Culture of cells for tissue engineering", Ed.: Vunjak-Novakovic G and Freshney RI, 2006

Livak, K.J. & Schmittgen T.D., Methods 25, 402-408, 2011

Smith M.M. & Little C.B. in "Osteoarthritis" Ed.: Moskowitz et al., Wolters Kluver, 2007.

Lahaye, M. & Robic, A. (2007) Structure and functiontional properties of ulvan, a polysaccharide from green seaweeds. Biomacromolecules, 8(6), 1765-1774.

Papy-Garcia, D., Barbier-Chassefiere, V., Rouet, V., Kerros, M-E., Klochendler, C., Tournaire, M-C., Barritault, D., Caruelle, J-P. & Petit, E. (2005) Nondegradative sulfation of polysaccharides. Synthesis and structure characterisation of biologically active heparin sulphate mimetics. Macromolecules, 38, 4647-4654.

Nagasawa, K., Inoue, Y. & Kamata, T. (1977) Solvolytic desulfation of glycosaminoglycuronan sulfates with dimethyl sulfoxide containing water or methanol. Carbohydrate Research, 58, 47-55.

Suzuki, K., Otani, J. & Tanaka, S. (2009) Angiogenesis inhibitor and use thereof. JP2009057285 (Konan Chemical Manufacturing C°, LTD)

## Claims

1. A viscosupplement composition comprising ulvan for use in the treatment, therapy or prophylaxis of a musculoskeletal disease.

2. The composition for use according to claim 1, wherein the sulphation degree of ulvan is 0.1% to 75%, preferably wherein the sulphation degree of ulvan is 0.1% to 6%.

3. The composition for use according to any of the preceding claim, wherein the ulvan has an average molecular weight between 100 and 10,000 kDa, preferably an average molecular weight between 100 and 600 kDa.

4. The composition for use according to any preceding claim, wherein the ulvan comprises 5% to 50% glucuronic acid, and 0 to 50% iduronic acid, in terms of the relative molar percentage of monomers, preferably wherein the ulvan comprises 25% to 50% glucuronic acid, and 0 to 15% iduronic acid, in terms of the relative molar percentage of monomers.

5. The composition for use according to any preceding claim, wherein the ulvan comprises 25% to 100% of the disaccharide unit [glucuronic acid-rhamnose sulphate] and 25% to 100% of the disaccharide unit [iduronic acid-rhamnose sulphate], in terms of the relative molar percentage of units, preferably , wherein the ulvan comprises 40% to 100% of the disaccharide unit [glucuronic acid-rhamnose sulphate] and 25% to 50% of the disaccharide unit [iduronic acid-rhamnose sulphate], in terms of the relative molar percentage of units .

6. The composition for use according to any preceding claim, wherein the ulvan comprises 25% to 100% of the A3S and 25% to 100% of the B3S repeating disaccharide unit, in terms of the relative molar percentage of units, preferably wherein the ulvan comprises 40% to 100% of the A3S and 25% to 50% of the B3S repeating disaccharide unit, in terms of the relative molar percentage of units, wherein A3S is the repeating disaccharide unit [>4-beta-D-glucuronic acid-(1>4)-alpha-L-rhamnose 3-sulphate-1>], and B3S is the repeating disaccharide unit [>4-alpha-L-iduronic acid-(1>4)-alpha-L-rhamnose 3-sulphate-1>].

7. The composition for use according to any preceding claim, wherein the ulvan comprises additional functional groups such as methacrylate, amine, aldehydes, itaconates, acrylamides, or acrylates.

8. The composition for use according to claim 7, wherein the degree of substitution of ulvan by functional groups is between 0.1% and 50%, preferably wherein the degree of substitution of ulvan by functional groups is between 0.1% and 30%

9. The composition for use according to any preceding claim, wherein the ulvan has a concentration of 0.1% to 30% w/V in the composition, preferably wherein the ulvan has a concentration of 0.4% to 12% w/V in the composition.

10. The composition for use according to any preceding claim, further comprising one or more sulphated polysaccharides in addition to the ulvan.

11. The composition for use according to the preceding claim, wherein the one or more sulphated polysaccharides are selected from the group consisting of gellan sulphate, chondroitin sulphate, keratan sulphate, heparin sulphate, dextran sulphate and xylose sulphate.

12. The composition for use according to any preceding claim, further comprising hyaluronic acid.

13. The composition for use according to any preceding claim, further comprising one or more sulphated polysaccharides in addition to the ulvan, and hyaluronic acid.

14. The composition for use according to any one of claims 1 to 12 for use in the treatment or prophylaxis of arthritis.

15. The composition for use according to any preceding claims, wherein the composition is administered by intra-articular injection.

16. Ulvan for use in the treatment, therapy or prophylaxis of a musculoskeletal disease such as arthritis.

**Patentansprüche**

1. Eine Viscosupplement-Zusammensetzung mit Ulvan zur Behandlung, Therapie oder Prophylaxe einer Erkrankung des Bewegungsapparats.

2. Die Zusammensetzung zur Verwendung nach Anspruch 1, wobei der Sulfatisierungsgrad von Ulvan 0,1 % bis 75 % beträgt, wobei der Sulfatisierungsgrad von Ulvan vorzugsweise 0,1 % bis 6 % beträgt.

3. Die Zusammensetzung zur Verwendung nach einem der vorhergehenden Ansprüche, wobei das Ulvan eine durchschnittliche molekulare Masse zwischen 100 und 10.000 kDa, vorzugsweise eine durchschnittliche molekulare Masse zwischen 100 und 600 kDa aufweist.

4. Die Zusammensetzung zur Verwendung nach einem der vorhergehenden Ansprüche, wobei das Ulvan 5 % bis 50 % Glucuronsäure und 0 bis 50 % Iduronsäure, bezogen auf den relativen Stoffmengenanteil an Monomeren, enthält, wobei das Ulvan vorzugsweise 25 % bis 50 % Glucuronsäure und 0 bis 15 % Iduronsäure, bezogen auf den relativen Stoffmengenanteil an Monomeren, enthält.

5. Die Zusammensetzung zur Verwendung nach einem der vorhergehenden Ansprüche, wobei das Ulvan 25 % bis 100 % der Disaccharid-Einheit [Glucuronsäure-Rhamnosesulfat] und 25 % bis 100 % der Disaccharid-Einheit [Iduronsäure-Rhamnosesulfat], bezogen auf den relativen Stoffmengenanteil der Einheiten, enthält, wobei das Ulvan vorzugsweise 40 % bis 100 % der Disaccharid-Einheit [Glucuronsäure-Rhamnosesulfat] und 25 % bis 50 % der Disaccharid-Einheit [Iduronsäure-Rhamnosesulfat], bezogen auf den relativen Stoffmengenanteil der Einheiten, enthält.

6. Die Zusammensetzung zur Verwendung nach einem der vorhergehenden Ansprüche, wobei das Ulvan 25 % bis 100 % der A3S- und 25 % bis 100 % der sich wiederholenden B3S-Disaccharid-Einheit, bezogen auf den relativen Stoffmengenanteil der Einheiten, enthält, wobei das Ulvan vorzugsweise 40 % bis 100 % der A3S- und 25 % bis 50 % der sich wiederholenden B3S-Disaccharid-Einheit, bezogen auf den relativen Stoffmengenanteil der Einheiten, enthält, wobei A3S die sich wiederholende Disaccharid-Einheit [>4-beta-D-Glucuronsäure-(1>4)-alpha-L-Rhamnose 3-Sulfat-1>] und B3S die sich wiederholende Disaccharid-Einheit [>4-alpha-L-Iduronsäure-(1>4)-alpha-L-Rhamnose 3-Sulfat-1>] ist.

7. Die Zusammensetzung zur Verwendung nach einem der vorhergehenden Ansprüche, wobei das Ulvan zusätzliche Funktionsgruppen wie Methacrylate, Amine, Aldehyde, Itaconate, Acrylamide oder Acrylate umfasst.

8. Die Zusammensetzung zur Verwendung nach Anspruch 7, wobei der Substitutionsgrad des Ulvans durch Funktionsgruppen zwischen 0,1 % und 50 % liegt, wobei der Substitutionsgrad des Ulvans durch Funktionsgruppen vorzugsweise zwischen 0,1 % und 30 % liegt.

9. Die Zusammensetzung zur Verwendung nach einem der vorhergehenden Ansprüche, wobei das Ulvan eine Konzentration von 0,1 % bis 30 % w/V in der Zusammensetzung aufweist, wobei das Ulvan vorzugsweise eine Konzentration von 0,4 % bis 12 % w/V in der Zusammensetzung aufweist.

10. Die Zusammensetzung zur Verwendung nach einem der vorhergehenden Ansprüche, ferner zusätzlich zu dem Ulvan umfassend ein oder mehrere sulfatierte Polysaccharide.

11. Die Zusammensetzung zur Verwendung nach dem vorhergehenden Anspruch, wobei das ein oder mehrere sulfatierten Polysaccharide aus einer Gruppe bestehend aus Gellansulfat, Chondroitinsulfat, Keratansulfat, Heparinsulfat, Dextransulfat und Xylosesulfat ausgewählt werden.

12. Die Zusammensetzung zur Verwendung nach einem der vorhergehenden Ansprüche, ferner umfassend Hyaluronsäure.

13. Die Zusammensetzung zur Verwendung nach einem der vorhergehenden Ansprüche, ferner zusätzlich zu dem Ulvan umfassend ein oder mehrere sulfatierte Polysaccharide und Hyaluronsäure.

14. Die Zusammensetzung zur Verwendung nach einem der Ansprüche 1 bis 12 zur Verwendung bei der Behandlung oder Prophylaxe von Arthritis.

15. Die Zusammensetzung zur Verwendung nach einem der vorhergehenden Ansprüche, wobei die Zusammensetzung durch intraartikuläre Injektion verabreicht wird.

16. Ulvan zur Verwendung bei der Behandlung, Therapie oder Prophylaxe einer Erkrankung des Bewegungsapparats wie Arthritis.

## Revendications

1. Une composition de viscosupplément comprenant des ulvanes pour le traitement, la thérapie ou la prophylaxie d'une maladie musculosquelettique.

2. La composition pour utiliser selon la revendication 1, dans laquelle le taux de sulfatation des ulvanes est de 0.1% à 75%, de préférence dans laquelle le taux de sulfatation des ulvanes est de 0.1% à 6%.

3. La composition pour utiliser selon l'une quelconque des revendications précédentes, dans laquelle l'ulvane a un poids moléculaire moyen compris entre 100 et 10,000 kDa, de préférence un poids moléculaire moyen compris entre 100 et 600 kDa.

4. La composition pour utiliser selon l'une quelconque des revendications précédentes, dans laquelle l'ulvane comprend 5% à 50% d'acide glucuronique, et 0 à 50% d'acide iduronique, en termes de pourcentage molaire relatif des monomères, de préférence dans laquelle l'ulvane comprend 25% à 50% d'acide glucuronique, et 0 à 15% d'acide iduronique, en termes de pourcentage molaire relatif des monomères.

5. La composition pour utiliser selon l'une quelconque des revendications précédentes, dans laquelle l'ulvane comprend 25% à 100% du motif disaccharide [acide glucuronique - rhamnose sulfaté] et 25% à 100% du motif disaccharide [acide iduronique - rhamnose sulfaté], en termes de pourcentage molaire relatif des motifs, de préférence, dans laquelle l'ulvane comprend 40% à 100% du motif disaccharide [acide glucuronique - rhamnose sulfaté] et 25% à 50% du motif disaccharide [acide iduronique - rhamnose sulfaté], en termes de pourcentage molaire relatif des motifs.

6. La composition pour utiliser selon l'une quelconque des revendications précédentes, dans laquelle l'ulvane comprend 25% à 100% du motif disaccharide répétitif A3S et 25% à 100% du B3S, en termes de pourcentage molaire relatif des motifs, de préférence dans laquelle l'ulvane comprend 40% à 100% du motif disaccharide répétitif A3S et 25% à 50% du B3S, en termes du pourcentage molaire relatif des motifs, dans laquelle A3S est le motif disaccharide répétitif [>4-beta-D-acide glucuronique-(1>4)-alpha-L-rhamnose-3-sulfate-1>], et B3S est le motif disaccharide répétitif [>4-alpha-L-acide iduronique-(1>4)-alpha-L-rhamnose-3-sulfate-1>].

7. La composition pour utiliser selon l'une quelconque des revendications précédentes, dans laquelle l'ulvane comprend des groupes fonctionnels supplémentaires tels que le méthacrylate, l'amine, les aldéhydes, les itaconates, les acrylamides, ou les acrylates.

8. La composition pour utiliser selon la revendication 7, dans laquelle le taux de substitution d'ulvanes par des groupes fonctionnels est situé entre 0.1% et 50%, de préférence dans laquelle le taux de substitution d'ulvanes par des groupes fonctionnels est situé entre 0.1% et 30%.

9. La composition pour utiliser selon l'une quelconque des revendications précédentes, dans laquelle l'ulvane a une concentration de 0.1% à 30% en poids par volume dans la composition, de préférence dans laquelle l'ulvane a une concentration de 0.4% à 12% en poids par volume dans la composition.

10. La composition pour utiliser selon l'une quelconque des revendications précédentes, comprenant également un ou plusieurs polysaccharides sulfatés en plus de l'ulvane.

11. La composition pour utiliser selon l'une quelconque des revendications précédentes, dans laquelle un ou plusieurs polysaccharides sulfatés sont sélectionnés à partir du groupe consistant en sulfate de gellane, sulfate de chondroïtine, sulfate de kératine, sulfate d'héparine, sulfate de dextran et sulfate de xylose.

12. La composition pour utiliser selon l'une quelconque des revendications précédentes, comprenant également de l'acide hyaluronique.

13. La composition pour utiliser selon l'une quelconque des revendications précédentes, comprenant également un ou plusieurs polysaccharides sulfatés en plus de l'ulvane, et de l'acide hyaluronique.

14. La composition pour utiliser selon l'une quelconque des revendications 1 à 12 pour utiliser dans le traitement ou la prophylaxie de l'arthrite.

15. La composition pour utiliser selon l'une quelconque des revendications précédentes, dans laquelle la composition est administrée par injection intra-articulaire.

16. Des ulvanes pour utiliser dans le traitement, la thérapie ou la prophylaxie d'une maladie musculosquelettique telle que l'arthrite.

**Figure 1**

$A_{3s}$

$B_{3s}$

$U_{3s}$

$U_{2's3s}$

**Figure 2**

**Figure 3**

**Figure 4**

**Figure 5**

**Figure 6**

Figure 7

Figure 8

Figure 9

| Normal Cartilage | OA Cartilage Before Treatment | OA Cartilage After Treatment |

Markedly reduced matrix staining

Lamination, fissures

Improved staining of matrix

Reduction of lamination

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009057285 B
- WO 2006073835 A
- WO 2011119059 A1

**Non-patent literature cited in the description**

- **ALVES, A. ; CARIDADE, S. G. ; MANO, J. F. ; SOUSA, R. A. ; REIS, R. L.** Extraction and physico-chemical characterization of a versatile biodegradable polysaccharide obtained from green algae. *Carbohydrate Research,* 2010, vol. 345, 2194-2200
- **BARROS, A. A. A. ; ALVES, A. ; NUNES, C. ; COIMBRA, M. A. ; PIRES, R. A. ; REIS, R. L.** Carboxymethylation of ulvan and chitosan and their use as polymeric components of bone cements. *Acta Biomaterialia,* 01 July 2013
- Isolation and Analysis of Cell Wall Polymers from Olive Pulp. **COIMBRA, M. A. ; DELGADILLO, I. ; WALDRON, K. W. ; SELVENDRAN, R. R.** Modern Methods of Plant Analysis. Springer-Verlag, 1996, 19-44
- **LAHAYE, M. ; INIZAN, F. ; VIGOUROUX, J.** NMR analysis of the chemical structure of ulvan and of ulvan-boron complex formation. *Carbohydrate Polymers,* 1998, vol. 36, 239-249
- **ROBIC, A. ; SASSI, J.-F. ; LAHAYE, M.** Impact of stabilization treatments of the green seaweed Ulva rotundata (Chlorophyta) on the extraction yield, the physico-chemical and rheological properties of ulvan. *Carbohydrate Polymers,* 2008, vol. 74 (3), 344-352
- **GONÇALVES, C. ; RODRIGUEZ-JASSO, R. M. ; GOMES, N. ; TEIXEIRA, J. A. ; BELO, I.** Adaptation of dinitrosalicylic acid method to microtiter plates. *Analytical Methods,* 2010, vol. 2 (12), 2046
- **SUBHAPRADHA N. ; SUMAN S. ; RAMASAMY P. ; SARAVANAN R. ; SHANMUGAN V ; SRINIVASAN A. ; SHANMUGAN A.** Anticoagulant and antioxidant activity of sulphated chitosan from the shell of donacid clam Donax scortum (Linnaeus, 1758). *International Journal of Nutrition, Pharmacology, Neurological Diseases,* 2013, vol. 3 (1), 39-45
- **HAMCERENCU, M. ; DESBRIERES, J. ; KHOUKH A. ; POPA M. ; RIESS G.** Synthesis and characterization of new unsaturated esters of gellan gum. *Carbohydrate Polymers,* 2008, vol. 71, 92-100
- **MASUDA, K ; SAH RL.** Culture of cells for tissue engineering. 2006
- **LIVAK, K.J. ; SCHMITTGEN T.D.** *Methods,* 2011, vol. 25, 402-408
- **SMITH M.M. ; LITTLE C.B. et al.** Osteoarthritis. Wolters Kluver, 2007
- **LAHAYE, M. ; ROBIC, A.** Structure and functiontional properties of ulvan, a polysaccharide from green seaweeds. *Biomacromolecules,* 2007, vol. 8 (6), 1765-1774
- **PAPY-GARCIA, D. ; BARBIER-CHASSEFIERE, V. ; ROUET, V. ; KERROS, M-E. ; KLOCHENDLER, C. ; TOURNAIRE, M-C. ; BARRITAULT, D. ; CARUELLE, J-P. ; PETIT, E.** Nondegradative sulfation of polysaccharides. Synthesis and structure characterisation of biologically active heparin sulphate mimetics. *Macromolecules,* 2005, vol. 38, 4647-4654
- **NAGASAWA, K. ; INOUE, Y. ; KAMATA, T.** Solvolytic desulfation of glycosaminoglycuronan sulfates with dimethyl sulfoxide containing water or methanol. *Carbohydrate Research,* 1977, vol. 58, 47-55